# EUROPEAN PATENT APPLICATION

(11) **EP 2 319 539 A1**
(43) Date of publication of application: **11.05.2011**
(21) Application number: 09800218.1
(22) Date of filing: 23.07.2009
(51) Int. Cl.: A61K 45/00, A61K 31/4409, A61K 31/4439, A61K 31/551, A61P 27/10, A61P 43/00, C07D 213/75, C07D 401/04, C07D 401/12

(54) **PROPHYLACTIC OR THERAPEUTIC AGENT FOR AXIAL MYOPIA**

(30) Priority: 24.07.2008 JP 2008190673
(71) Applicant: Osaka University, Suita-shi Osaka 565-0871 (JP)
(72) Inventor: IKUNO, Yasushi, Suita-shi Osaka 565-0871 (JP); TANO, Yasuo, Deceased (JP)
(74) Representative: Kalhammer, Georg
(86) International application number: PCT/JP2009/003455
(87) International publication number: WO 2010/010702

(57) **Abstract**

Since high myopia increases the risks of retinal detachment, myopic chorioretinal atrophy, myopic choroidal neovascularization, glaucoma, and cataract, establishment of prophylaxis against the progress of myopia is desired. However, at present, there is no effective prophylaxis or therapy for axial myopia, which is considered to cause high myopia. The present invention, which was completed based on the finding that Rho kinase inhibitors have an inhibitory action on axial length extension, provides a prominently effective prophylactic or therapeutic agent comprising a Rho kinase inhibitor as an active ingredient for axial myopia.

## Description

### TECHNICAL FIELD

The present invention relates to a prophylactic or therapeutic agent for axial myopia. In more detail, the present invention relates to a prophylactic or therapeutic agent for axial myopia comprising a Rho kinase inhibitor as an active ingredient.

### BACKGROUND ART

Myopia is a condition in which light passing through the cornea forms an image not on but before the retina, and such an image cannot be clearly captured. Myopia is classified roughly into two categories, accommodative myopia and axial myopia. Accommodative myopia is assumed to be related to accommodation in prolonged near work, that is, increased refractivity of the lens caused by increased contraction of the ciliary muscle. Normally, when near work is discontinued, the ciliary muscle is relaxed and accommodation is released. However, the contraction state resulting from prolonged near work sometimes remains fixed, and thus myopia manifests itself. Such a myopic condition is called accommodative myopia. In contrast, in axial myopia, light from infinity forms an image before the retina due to increased axial length (length from the cornea to the retina). It is believed that high myopia is caused by axial length extension, and from various experiments, both the retina and the central nervous system are considered to be involved in axial length extension (see Non Patent Literature 1).

To prevent the progress of myopia, ophthalmic instillation of tropicamide (Mydrin-M (registered trademark)) has been attempted as a method to improve the strain of the ciliary muscle based on a mechanism in which refractive myopia is caused by increased contraction of the ciliary muscle. However, the effect is not statistically significant (see Non Patent Literature 1). Atropine blocks accommodation by its ciliary muscle relaxing action, suppresses axial length extension in animal experiments, releases dopamine in retinal cells to inhibit eye growth, and suppresses growth hormone secretion from the pituitary gland to inhibit the growth of the normal eye. Through these actions, atropine is apparently capable of inhibiting the progress of myopia. Although statistically significant inhibition of the progress of myopia has been reported in Taiwan, atropine has problems of medium and short term side effects, such as contact dermatitis, photophobia resulting from mydriasis, blurred vision, and adverse effects on the retina and the lens due to ultraviolet light exposure (see Non Patent Literature 1). Pirenzepine prevented form-deprivation myopia by suppression of axial length extension at an animal experiment level (see Non Patent Literature 1). However, in a 2-year clinical trial of children aged 8 to 12 conducted in the U.S., while a significant difference in accommodative myopia (refraction of the lens) was shown, no significant difference in axial length was found (see Non Patent Literature 2).

Patent Literature 1 discloses an axial length controlling agent comprising a transforming growth factor controlling substance as an active ingredient, but the agent has not yet been in clinical use. Thus, there are some agents which suppress axial length extension at an animal experiment level, but none of such agents, so far as known, have any clinically validated effectiveness in axial myopia.

Rho kinase is an intracellular serine/threonine phosphorylation enzyme identified as a target protein of a small GTP-binding protein Rho. Rho kinase has 2 isoforms: ROCα (Rho kinaseα, also referred to as ROCK-II) and ROCβ (Rho kinaseβ, also referred to as ROCK-I). These isoforms are ubiquitously expressed in the body, but ROCα is preferentially expressed in the brain and skeletal muscle.

It has become clear that Rho kinase is deeply involved in cellular physiological functions, such as contraction, proliferation, migration, and gene expression induction, and from examinations in animal models and in humans to date, it is known that Rho kinase is deeply involved in the clinical conditions of various diseases centering on cardiovascular diseases. Therefore, it is suggested that Rho kinase inhibitors are useful for the therapy of these diseases. Potential as a therapeutic agent for, in particular, diseases resulting from excessive contraction of vascular smooth muscle (coronary spasm, cerebrovascular spasm, hypertension, pulmonary hypertension, sudden death, or the like), arteriosclerotic diseases (angina pectoris, myocardial infarction, restenosis, cerebral infarction, hypertensive vascular disease, cardiac failure, or the like), osteoporosis, erectile dysfunction, renal disease, cancer, insulin resistance, bronchial asthma, glaucoma, or the like, is suggested (see Non Patent Literature 3 and Patent Literature 2). Actually, a liquid for intravenous-drip comprising fasudil, which is a Rho kinase inhibitor, and having efficacy of improving cerebrovascular spasm after subarachnoid bleeding and accompanying cerebral ischemia is approved as a medicine and currently in clinical use in Japan.

It is suggested that Rho kinase inhibitors are useful also in the field of ophthalmology and clinical application of Rho kinase inhibitors as a therapeutic agent for glaucoma for example is being investigated. Patent Literature 3 discloses a prophylactic or therapeutic agent for glaucoma comprising a compound which has a Rho kinase inhibitory action. The disclosure is based on the finding that Rho kinase inhibitors have actions of lowering intraocular pressure, improving optic disc blood flow, and accelerating aqueous outflow. Patent Literature 4 discloses a therapeutic agent for glaucoma comprising a combination of a Rho kinase inhibitor and prostaglandins, and Patent Literature 5 discloses a therapeutic agent for glaucoma comprising a combination of a Rho kinase inhibitor and a beta blocker. Patent Literature 6 discloses a prophylactic or therapeutic agent for eyestrain and a prophylactic or therapeutic agent for false myopia, both comprising a compound which has a Rho kinase inhibitory action. The disclosure is based on the finding that Rho kinase inhibitors suppress contraction of the ciliary muscle and are useful as a prophylactic or therapeutic agent for eyestrain and false myopia caused by persistent abnormal tense of the ciliary muscle.

However, none of the above-mentioned actions of lowering intraocular pressure, improving optic disc blood flow, accelerating aqueous outflow, and suppressing contraction of the ciliary muscle suggest suppression of axial length extension, and even those skilled in the art could not have predicted that Rho kinase inhibitors are useful for prophylaxis or therapy of axial myopia.

### CITATION LIST

### [Patent Literatures]

[Patent Literature 1]: JP 09-301891 A
[Patent Literature 2]: WO 98/06433
[Patent Literature 3]: WO 00/09162
[Patent Literature 4]: JP 2004-107335 A
[Patent Literature 5]: JP 2004-182723 A
[Patent Literature 6]: JP 2004-277432 A

### [Non Patent Literatures]

[Non Patent Literature 1]: Kenichi Yoshino, "Gakudouji no kinshi chiryou ni tsuite (Therapy for myopic school-age children)." Chiryou [The journal of therapy], special August number (Vol. 87), 1388-1392 (2005)
[Non Patent Literature 2]: Siatkowski RM, Cotter SA, Crockett RS, Miller JM, Novack GD, Zadnik K; U. S. Pirenzepine Study Group. "Two-year multicenter randomized, double-masked, placebo-controlled, parallel safety and efficacy study of 2% pirenzepine ophthalmic gel in children with myopia." J AAPOS. In Press Corrected Proof, Available online 25 March 2008
[Non Patent Literature 3]: Shunsuke Tawara, Hiroaki Shimokawa, "Progress of the study of Rho kinase and future perspective of the inhibitor." YAKUGAKU ZASSHI, 127(3), 501-514 (2007)

### SUMMARY OF INVENTION

### TECHNICAL PROBLEM

Advancement of glasses, various kinds of contact lenses, and refractive surgery has increased options in myopia correction. However, myopia is mostly bilateral and irreversibly progressive, and affects socially active age groups. Therefore, myopia itself decreases quality of life (QOL). From medical point of view, increased risks of retinal detachment, myopic chorioretinal atrophy, myopic choroidal neovascularization, glaucoma, and cataract, which accompany high myopia, can cause more serious ocular complications. For addressing such concerns, what is desired is establishment of prophylaxis against the progress of school-age myopia, which is highly progressive, and of adult myopia (see Non Patent Literature 1).

In particular, for axial myopia, which is considered to cause high myopia, there is no effective prophylaxis or therapy at present, and it is expected that achieving prophylaxis or therapy of axial myopia not only improves QOL but also decreases the risks of developing the above-mentioned serious ocular diseases. For this reason, development of a clinically effective prophylactic or therapeutic agent for axial myopia is strongly desired.

In these surroundings, an object of the present invention is to provide a prominently effective prophylactic or therapeutic agent for axial myopia.

### SOLUTION TO PROBLEM

The present inventors made extensive examination to solve the problem described above. As a result, they found that Rho kinase inhibitors have an inhibitory action on axial length extension, and that because of the action, a Rho kinase inhibitor can be an excellent active ingredient for a prophylactic or therapeutic agent for axial myopia, and finally completed the present invention.

That is, the present invention relates to a prophylactic or therapeutic agent for axial myopia, and includes the following.
[1] A prophylactic or therapeutic agent for axial myopia comprising a Rho kinase inhibitor as an active ingredient.
[2] The prophylactic or therapeutic agent for axial myopia according to the above [1], wherein the Rho kinase inhibitor is a compound selected from the group consisting of (R)-(+)-trans-N-(4-pyridyl)-4-(1-aminoethyl)-cyclohexanecarboxamide, 1-(5-isoquinolinesulfonyl)homopiperazine, 1-(1-hydroxy-5-isoquinolinesulfonyl)homopiperazine, (S)-(+)-2-methyl-1-[(4-methyl-5-isoquinolinyl)sulfonyl]-homopiperazine, (S)-(+)-4-glycyl-2-methyl-1-[(4-methyl-5-isoquinolinyl)-sulfonyl]homopiperazine, N-(4-pyridyl)-N'-(2,4,6-trichlorophenyl)urea, and 3-(4-pyridyl)-1H-indole, or a salt thereof.
[3] The prophylactic or therapeutic agent for axial myopia according to the above [1] or [2], which is for ophthalmic instillation.
[4] The prophylactic or therapeutic agent for axial myopia according to the above [1] or [2], which is for intravitreal administration.

### ADVANTAGEOUS EFFECTS OF INVENTION

The prophylactic or therapeutic agent of the present invention for axial myopia comprises a Rho kinase inhibitor as an active ingredient, which has an inhibitory action on axial length extension, and therefore is extremely useful for prophylaxis or therapy of axial myopia.

### DESCRIPTION OF EMBODIMENTS

The present invention relates to a prophylactic or therapeutic agent for axial myopia comprising a Rho-kinase inhibitor as an active ingredient. As used herein, the Rho kinase inhibitor means a compound which inhibits the action of serine/threonine kinase activated with the activation of a small GTP-binding protein Rho. In addition, as used herein, axial myopia means a myopic condition in which, due to the extension of the axial length (length from the cornea to the retina), light passing through the cornea forms an image not on but before the retina, and such an image cannot be clearly captured.

The inventors found that intravitreal administration of a Rho kinase inhibitor to a chicken model of form-deprivation myopia significantly suppresses axial length extension. This finding revealed for the first time that Rho kinase is involved in axial length extension, i.e., Rho kinase is involved in the onset of axial myopia, and that Rho kinase inhibitors are extremely useful for prophylaxis or therapy of axial myopia.

The Rho kinase inhibitor used for the prophylactic or therapeutic agent of the present invention for axial myopia is not particularly limited and may be any compound that inhibits the action of Rho kinase. Examples of the Rho kinase inhibitor preferably used for the prophylactic or therapeutic agent of the present invention include (R)-(+)-trans-N-(4-pyridyl)-4-(1-aminoethyl)-cyclohexanecarboxamide, 1-(5-isoquinolinesulfonyl)homopiperazine, 1-(1-hydroxy-5-isoquinolinesulfonyl)homopiperazine, (S)-(+)-2-methyl-1-[(4-methyl-5-isoquinolinyl)sulfonyl]-homopiperazine, (S)-(+)-4-glycyl-2-methyl-1-[(4-methyl-5-isoquinolinyl)-sulfonyl]homopiperazine, N-(4-pyridyl)-N'-(2,4,6-trichlorophenyl)urea, and 3-(4-pyridyl)-1H-indole.

(R)-(+)-trans-N-(4-pyridyl)-4-(1-aminoethyl)-cyclohexanecarboxamide is also referred to as Y27632. This compound has the structure represented by the formula (I) shown below, and is known as a strong selective inhibitor of ROCK-I and ROCK-II (Reference: M. Uehata, et al. "Calcium sensitization of smooth muscle mediated by a Rho-associated protein kinase in hypertension." Nature 389, 990 (1997)).

1-(5-isoquinolinesulfonyl)homopiperazine is also referred to as fasudil or HA1077. This compound has the structure represented by the formula (II) shown below, and is approved in Japan as an active ingredient used for a medicine to improve cerebrovascular spasm after subarachnoid bleeding and accompanying cerebral ischemia (Reference: M. Shirotani, et al. "A new type of vasodilator, HA1077, an isoquinoline derivative, inhibits proliferation of bovine vascular smooth muscle cells in culture." J. Pharmacol. Exp. Ther. 259, 738 (1991)).

1-(1-hydroxy-5-isoquinolinesulfonyl)homopiperazine is also referred to as hydroxyfasudil or HA1100. This compound is an active metabolite of fasudil and has the structure represented by the formula (III) shown below (Reference: H. Shimokawa, et al. "Rho kinase-mediated pathway induces enhanced myosin light chain phosphorylations in a swine model of coronary artery spasm." Cardiovasc. Res. 43, 1029 (1999)).

(S)-(+)-2-methyl-1-[(4-methyl-5-isoquinolinyl)-sulfonyl]homopiperazine is also referred to as H1152. This compound is a fasudil derivative highly selective for ROCK II, and has the structure represented by the formula (IV) shown below (Reference: M. Ikenoya, et al. "Inhibition of rho-kinase-induced myristoylated alanine-rich C kinase substrate (MARCKS) phosphorylation in human neuronal cells by H-1152, a novel and specific Rho-kinase inhibitor." J. Neurochem. 81, 9 (2002)).

(S)-(+)-4-glycyl-2-methyl-1-[(4-methyl-5-isoquinolinyl)sulfonyl] homopiperazine is a glycyl-substitution product of the above H1152, and is also referred to as glycyl-H1152. This compound has the structure represented by the formula (V) shown below, and has a ROCK-II selectivity higher than that of H1152 (Tamura et al. "Development of specific Rho-kinase inhibitors and their clinical application." Biochim. Biophys. Acta 1754, 245 (2005)).

N-(4-pyridyl)-N'-(2,4,6-trichlorophenyl)urea has the structure represented by the formula (VI) shown below (A. Takami, et al. "Design and synthesis of Rho kinase inhibitors (I)." Bioorg. Med. Chem. 12, 2115 (2004)).

3-(4-pyridyl)-1H-indole has the structure represented by the formula (VII) shown below (J. C. Yarrow, et al. "Screening for cell migration inhibitors via automated microscopy reveals a Rho-kinase inhibitor." Chem. Biol. 12, 385 (2005)).

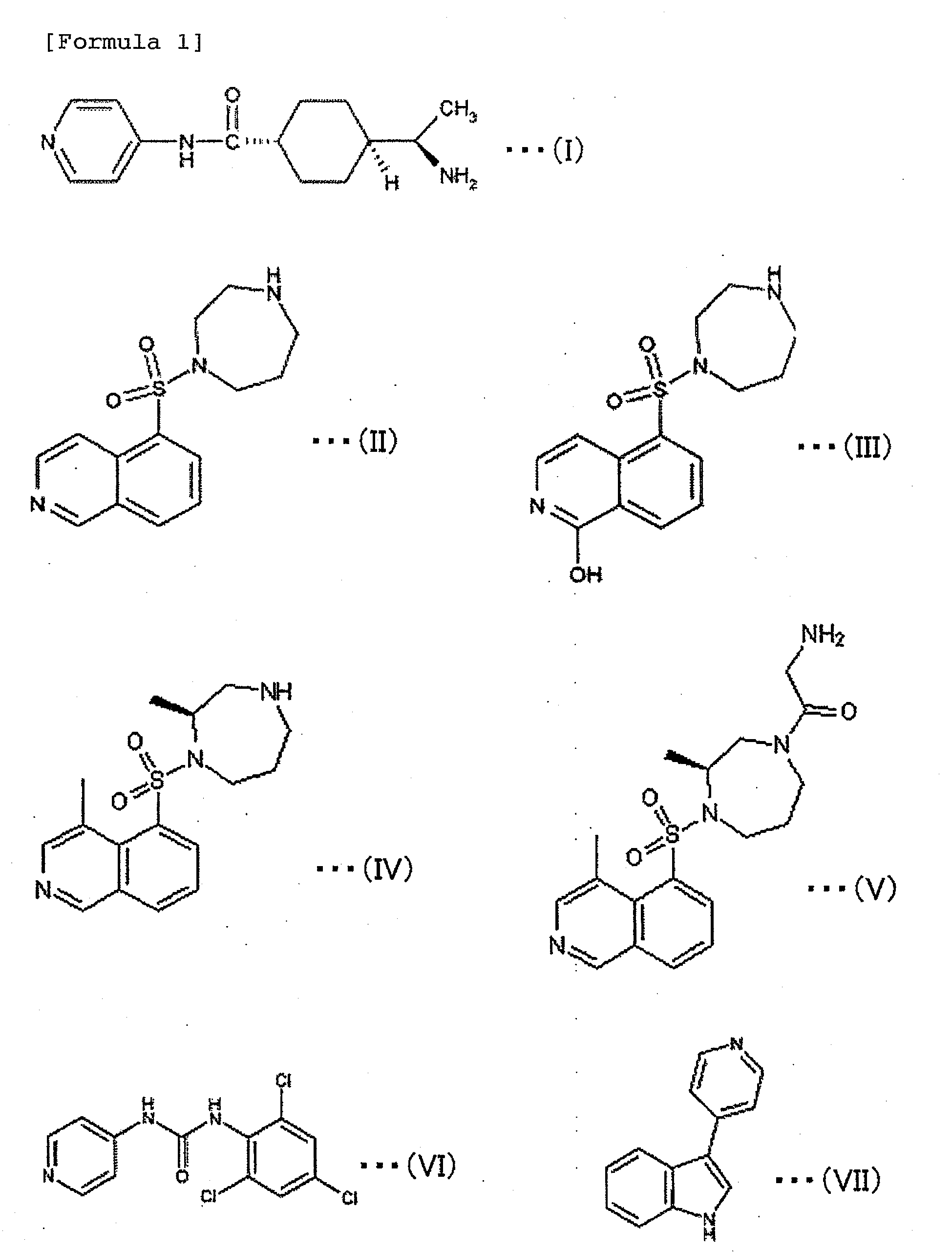

The above-mentioned compounds (the above formulae (I) to (VII)) exemplified as the Rho kinase inhibitor preferably used for the prophylactic or therapeutic agent of the present invention for axial myopia may be used in the form of a pharmacologically acceptable salt thereof. Examples of such a salt include salts with a base, such as an inorganic base and an organic base; and acid addition salts with an inorganic acid, an organic acid, a basic amino acid, and an acidic amino acid. Examples of the inorganic base include, alkali metals, such as sodium and potassium; alkaline earth metals, such as calcium and magnesium; aluminum, ammonium; and the like. Examples of the organic base include primary amines, such as ethanolamine; secondary amines, such as diethylamine, diethanolamine, dicyclohexylamine, and N,N'-dibenzylethylenediamine; and tertiary amines, such as trimethylamine, triethylamine, pyridine, picoline, and triethanolamine. Examples of the inorganic acid include hydrochloric acid, hydrobromic acid, nitric acid, sulfuric acid, and phosphoric acid. Examples of the organic acid include formic acid, acetic acid, trifluoroacetic acid, fumaric acid, oxalic acid, tartaric acid, maleic acid, citric acid, succinic acid, malic acid, methanesulfonic acid, benzenesulfonic acid, and p-toluenesulfonic acid. Examples of the basic amino acid include arginine, lysine, and ornithine. Examples of the acidic amino acid include aspartic acid and glutamic acid.

The compounds represented by the above formulae (I) to (VII) are publicly known Rho kinase inhibitors, and each compound or a salt thereof is commercially available. Also, the compounds can be readily produced by reference to related literature exemplified above.

The prophylactic or therapeutic agent of the present invention for axial myopia can be produced by, for example, mixing a Rho kinase inhibitor or a salt thereof and a pharmaceutically acceptable carrier. Examples of the pharmaceutically acceptable carrier used for solid preparations include, excipients (for example, lactose, cornstarch, mannitol, crystalline cellulose, or the like), lubricants (for example, magnesium stearate, talc, polyethylene glycol, colloidal silica, or the like), binders (syrup, gum arabic, gelatin, tragacanth gum, polyvinylpyrrolidone, or the like), and disintegrants (for example, potato starch, carboxymethylcellulose calcium, croscarmellose sodium, chitin, chitosan, or the like). Examples of the pharmaceutically acceptable carrier used for liquid preparations include, nonaqueous vehicles (for example, alcohols, such as ethanol, propylene glycol, and glycerol; oils and fats, such as olive oil, almond oil, sesame oil, cotton seed oil, castor oil, and corn oil; oily esters; or the like), solubilizing agents (for example, polyvinyl pyrrolidone, cyclodextrin, caffeine, polyethylene glycol, or the like), suspending agents (for example, surfactants, such as stearyl triethanolamine, sodium lauryl sulfate, and polysorbate 80; hydrophilic macromolecules, such as hydroxyethyl cellulose, carboxymethylcellulose, gelatin, and sorbitol syrup; or the like), thickeners (for example, yolk lecithin, gelatin, gum arabic, tragacanth gum, methyl cellulose, pectin, or the like), tonicity agents (for example, sorbitol, glycerol, polyethylene glycol, glucose, sodium chloride, or the like), emulsifiers (for example, lecithin, sorbitan monooleate, or the like), buffering agents (for example, phosphate buffering agent, borate buffering agent, citrate buffering agent, tartarate buffering agent, acetate buffering agent, or the like), and soothing agents (for example, benzyl alcohol or the like). These carriers can be appropriately blended into the prophylactic or therapeutic agent. In addition, if needed, preservatives (for example, p-hydroxybenzoate esters, benzalkonium chloride, chlorobutanol, or the like), chelating agents (for example, disodium edetate, sodium citrate, condensed sodium phosphate, or the like), antioxidant agents (for example, nitrite, ascorbic acid, cysteine, or the like), coloring agents (for example, tar dye, glycyrrhiza extract, riboflavin, or the like), sweetening agents (glucose, sucrose, saccharin, or the like), flavoring agents (for example, vanillin, menthol, or the like), and aromatic agents (for example, fennel oil, menthol, or the like) may be added by a conventional method. Examples of the pharmaceutically acceptable carrier include, in addition to those mentioned above, agar, casein, and collagen.

The prophylactic or therapeutic agent of the present invention for axial myopia is administered either orally or parenterally. Examples of the preparations for oral administration include solid preparations, such as a tablet, a powder, a granule, and a capsule; and liquid preparations, such as an emulsion, a syrup, and a suspension. Examples of the preparations for parenteral administration include an injection, an ointment, and an ophthalmic solution. The prophylactic or therapeutic agent of the present invention for axial myopia is preferably used in a form for local administration to the eye in terms of efficacy. Examples of the preparations for local administration to the eye include an ophthalmic solution, an injection (solution for intravitreal injection, subconjunctival injection, or sub-Tenon injection), an ophthalmic ointment, and an ophthalmic gel. The prophylactic or therapeutic agent of the present invention for axial myopia is preferably in the form of an ophthalmic solution or a solution for intravitreal injection. These preparations are preferably sustained-release preparations. The reason is that use of a sustained-release preparation enables extension of administration interval and reduction of administration frequency. In particular, since frequent injection to the eye increases the risk of complications, a sustained-release preparation is preferred as an injection. Sustained-release preparations can be produced by a publicly known method. Specific example of the sustained-release preparation include preparations comprising drug-containing particles of, for example, a biodegradable polymer (polylactic acid, or the like), a natural polymer (collagen, hyaluronic acid, or the like), and a synthetic polymer (methacrylic acid copolymer, or the like).

Injections can be obtained as an aqueous injection or an oily injection. An aqueous injection can be produced by dissolving a Rho kinase inhibitor or a salt thereof, with the above-described preservative, tonicity agent, solubilizing agent, or the like, in water for injection. An oily injection can be produced by dissolving or suspending a Rho kinase inhibitor or a salt thereof in propylene glycol, olive oil, sesame oil, cotton seed oil, or the like.

An aqueous ophthalmic solution can be produced by heating purified water, dissolving a preservative therein, adding a solubilizing agent, subsequently adding a Rho kinase inhibitor or a salt thereof, and making them completely dissolved. A buffering agent, a tonicity agent, a chelating agent, a thickener, or the like may be used as needed.

An aqueous suspended ophthalmic solution can be produced with use of, in addition to the additives used for aqueous ophthalmic solutions, a suspending agent suitably selected from the above-mentioned suspending agents. The aqueous ophthalmic solution and the aqueous suspended ophthalmic solution preferably have a pH value adjusted within the range usually employed in ophthalmic instillation, usually 4 to 9, and preferably 5 to 8.

A nonaqueous ophthalmic solution can be produced by dissolving or suspending a Rho kinase inhibitor or a salt thereof in a water- miscible solvent, such as an alcohol (for example, ethanol, ethylene glycol, macrogol, propylene glycol, glycerol, or the like); or in an oily solvent, such as an oil or fat (for example, olive oil, sesame oil, peanut oil, cotton seed oil, castor oil, corn oil, or the like).

An ophthalmic ointment can be produced by appropriately selecting and using as a base, for example, Vaseline, Plastibase, liquid paraffin, or the like.

An ophthalmic gel can be produced by appropriately selecting and using as a base, for example, carboxyvinyl polymer, ethylene maleic anhydride polymer, polyoxyethylene-polyoxypropylene block copolymer, gellan gum, or the like.

The amount of the active ingredient in the prophylactic or therapeutic agent of the present invention for axial myopia is 0.0001 to 100% by weight, preferably 0. 001 to 50 % by weight relative to the weight of the pharmaceutical preparation. The dose and the frequency of administration vary with the age, body weight, and dosage form, but in the case where the preparation is used for an adult as an ophthalmic solution, several drops at a time, preferably 1 to 3 drops at a time of a preparation containing 0.0001 to 10 w/v%, preferably 0.001 to 1 w/v% of a Rho-kinase inhibitor, can be administered several times a day, preferably 1 to 6 times a day; and in the case where the preparation is used for an adult as a solution for intravitreal injection, a preparation containing 0.0001 to 10 w/w%, preferably 0.001 to 1 w/w% of a Rho-kinase inhibitor, can be administered once every 4 to 8 weeks.

### EXAMPLES

Hereinafter, the invention will be more specifically described by referring to the examples below, but is not limited thereto.

### Example 1: Axial length suppression test with use of chicken model of myopia (I)

### (1) Chicken model of form-deprivation myopia

Chicks (white leghorn) 6 days of age were obtained, and one eye of each chick was covered with a mask hand-made of vinyl tape. The right eye of every chick was covered, and the left eye was used as a control. The chicks were kept under usual breeding conditions.

### (2) Rho kinase inhibitor

As a Rho kinase inhibitor, Y27632 dihydrochloride (made by Wako Pure Chemical Industries; see the above formula (I); hereinafter, abbreviated to "Y27632") and hydroxyfasudil hydrochloride (made by SIGMA; see the above formula (III); hereinafter, abbreviated to "hydroxyfasudil") were used. Y27632 was prepared at 50 µM in saline. Hydroxyfasudil was prepared at 1 mM in saline. To the control, saline was administered.

### (3) Administration method

Into the vitreous chambers of the chicks to which myopia was being induced by the mask, Y27632 or hydroxyfasudil was injected once a week (every 7 days). Specifically, to the chicks under anesthesia with Ketalar (registered trademark) and Selactar (registered trademark), injection to a site about 2 mm off the corneal ring was performed with use of a syringe for insulin injection.

To the Y27632 treatment group, 50 µL of the 50 µM Y27632 solution was intravitreally injected. Since the chick eyeball volume is about 1 mL, the final concentration of Y27632 in the eye was about 2.5 µM. To a control group not treated with Y27632, 50 µL of saline was intravitreally injected.

To the hydroxyfasudil treatment group, 20 µL of a diluted hydroxyfasudil solution prepared by diluting 1 µL of the 1 mM hydroxyfasudil solution to 20 µL was intravitreally injected. Since the chick eyeball volume is about 1 mL, the final concentration of hydroxyfasudil in the eye was about 1 µM. To a control group not treated with hydroxyfasudil, 20 µL of saline was intravitreally injected.

### (4) Measurement of eyeball

Four weeks after the induction of myopia, the chicks were sacrificed. The eyeballs were enucleated and the right eyeball and the left eyeball were measured for the size with a caliper. The degree of axial myopia was evaluated based on the axial length and the greatest transverse diameter. The differences of the axial length and the greatest transverse diameter between the right eye and the left eye were calculated to indicate the degree of myopia. Between the groups treated with the above-mentioned drugs and the respective control groups, the degree of myopia induction was compared.

### (5) Results

The results of the Y27632 treatment group and the control group are shown in Table 1. Table 1 clearly shows that the axial length and the greatest transverse diameter of the Y27632 treatment group were significantly reduced as compared to those of the control group.

**Table 1**

| | | Average right-left difference (mm) | Standard deviation (mm) |
|---|---|---|---|
| Y27632 treatment group (N=4) | | | |
| | Axial length | 0.26** | 0.09 |
| | Greatest transverse diameter | 0.29** | 0.06 |
| Saline treatment (control) group (N=3) | | | |
| | Axial length | 0.80 | 0.17 |
| | Greatest transverse diameter | 2.26 | 0.70 |

| | | | |
|---|---|---|---|
| **Significantly decreased as compared to the control (P<0.01) | | | |

The results of the hydroxyfasudil treatment group and the control group are shown in Table 2. Table 2 clearly shows that the axial length and the greatest transverse diameter of the hydroxyfasudil treatment group were significantly reduced as compared to those of the control group.

**Table 2**

| | | Average right-left difference (mm) | Standard deviation (mm) |
|---|---|---|---|
| Hydroxyfasudil treatment group (N=7) | | | |
| | Axial length | 0.37* | 0.65 |
| | Greatest transverse diameter | 0.48* | 0.87 |
| Saline treatment (control) group (N=11) | | | |
| | Axial length | 1.21 | 0.69 |
| | Greatest transverse diameter | 1.60 | 0.94 |

| | | | |
|---|---|---|---|
| *Significantly decreased as compared to the control (P<0.05) | | | |

As described above, since the Rho kinase inhibitors significantly inhibited the progress of axial myopia in chick models, it is clearly shown that Rho kinase is involved in axial length extension.

### Example 2: Axial length suppression test with use of chicken model of myopia (II)

### (1) Chicken model of form-deprivation myopia

Chicken models of form-deprivation myopia were made in the same manner as in Example 1. That is, chicks (white leghorn) 6 days of age were obtained, and one eye of each chick was covered with a mask hand-made of vinyl tape. The right eye of every chick was covered, and the left eye was used as a control. The chicks were kept under usual breeding conditions.

### (2) Rho kinase inhibitor

As a Rho kinase inhibitor, hydroxyfasudil hydrochloride (made by SIGMA; see the above formula (III); hereinafter, abbreviated to "hydroxyfasudil") was used. Hydroxyfasudil was dissolved in BSS Plus (registered trademark, made by Alcon) so that the concentration was 10 mM, and 50 µL of this solution was diluted with saline to 1000 µL. A hydroxyfasudil solution at a concentration of 500 nmol/1000 µL was thus prepared.

### (3) Administration method

To chicks to which myopia was being induced by the mask, hydroxyfasudil was instilled once daily for 4 weeks. Specifically, 1 drop (about 5 µL) of the above-mentioned hydroxyfasudil solution was instilled with use of a syringe with needle for insulin subcutaneous administration, "Low Dose" 30G (made by Becton, Dickinson and Company). The instillation was performed to the right eye and the left eye.

To the control, BSS Plus was intravitreally injected once a week (every 7 days). Specifically, the intravitreal injection was performed to a superotemporal site about 3 mm off the corneal ring avoiding cartilage tissue of the lens and sclera with use of the above-mentioned "Low Dose". The injection to the right eye and the left eye was performed 4 times in total.

### (4) Measurement of eyeball

Four weeks after the induction of myopia, the chicks were sacrificed. The eyeballs were enucleated and the right eyeballs and the left eyeballs were measured for the size with a caliper. The degree of axial myopia was evaluated based on the axial length and the greatest transverse diameter. The differences of the axial length and the greatest transverse diameter between the right eye and the left eye were calculated to indicate the degree of myopia. Between the group treated with hydroxyfasudil and the control group, the degree of myopia induction was compared.

### (5) Results

The results are shown in Table 3. Table 3 clearly shows that the axial length and the greatest transverse diameter of the hydroxyfasudil instillation group were significantly reduced as compared to those of the control group.

**Table 3**

| | | Average right-left difference (mm) | Standard deviation (mm) |
|---|---|---|---|
| Hydroxyfasudil treatment group (N=10) | | | |
| | Axial length | 0.61* | 0.55 |
| | Greatest transverse diameter | 0.91* | 0.53 |
| Saline treatment (control) group (N=7) | | | |
| | Axial length | 1.20 | 0.85 |
| | Greatest transverse diameter | 1.50 | 0.46 |

| | | | |
|---|---|---|---|
| *Significantly decreased as compared to the control (P<0.05, t-test) | | | |

### Example 3: Production Example

### Formulation 1: Ophthalmic solution

In sterile purified water, Y27632 dihydrochloride ((R)-(+)-trans-N-(4-pyridyl)-4-(1-aminoethyl)-cyclohexaneca rboxamide 2HCl), sodium dihydrogen phosphate dihydrate, and sodium chloride were dissolved. After the pH was adjusted to 7 with sodium hydroxide, sterile purified water was added until the whole volume reached 100 mL. An ophthalmic solution of the following composition was thus prepared.
In 100 ml
- Y27632 dihydrochloride 0.01 g
- Sodium dihydrogen phosphate dihydrate 0.1 g
- Sodium chloride 0.9 g
- Sodium hydroxide q.s.
- Sterile purified water q.s.

In the same manner as in the above Formulation 1, ophthalmic solutions containing 0.1 g or 0.001 g of Y27632 dihydrochloride per 100 mL can be prepared.

### Formulation 2: Ophthalmic solution

In sterile purified water, hydroxyfasudil hydrochloride (1-(1-hydroxy-5-isoquinolinesulfonyl)homopiperazine HCl), sodium dihydrogen phosphate dihydrate, and sodium chloride were dissolved. After the pH was adjusted to 7 with sodium hydroxide, sterile purified water was added until the whole volume reached 100 mL. An ophthalmic solution of the following composition was thus prepared.
In 100 ml
- Hydroxyfasudil hydrochloride 0.01 g
- Sodium dihydrogen phosphate dihydrate 0.1 g
- Sodium chloride 0.9 g
- Sodium hydroxide q.s.
- Sterile purified water q.s.

In the same manner as in the above Formulation 2, ophthalmic solutions containing 0.1 g or 0.001 g of hydroxyfasudil hydrochloride per 100 mL can be prepared.

### Formulation 3: Injection

In sterile purified water, Y27632 dihydrochloride ((R)-(+)-trans-N-(4-pyridyl)-4-(1-aminoethyl)-cyclohexaneca rboxamide 2HCl) and sodium chloride were dissolved. Sterile purified water was added until the whole volume reached 100 mL. An injection of the following composition was thus prepared. In 100 ml
- Y27632 dihydrochloride 0.001 g
- Sodium chloride 0.9 g
- Sterile purified water q.s.

In the same manner as in the above Formulation 3, injections containing 0.01 g or 0.0001 g of Y27632 dihydrochloride per 100 mL can be prepared.

### Formulation 4: Injection

In sterile purified water, hydroxyfasudil hydrochloride (1-(1-hydroxy-5-isoquinolinesulfonyl)homopiperazine HCl) and sodium chloride were dissolved. Sterile purified water was added until the whole volume reached 100 mL. An injection of the following composition was thus prepared.

### In 100 mL

- Hydroxyfasudil hydrochloride 0.001 g
- Sodium chloride 0.9 g
- Sterile purified water q.s.

In the same manner as in the above Formulation 4, injections containing 0.01 g or 0.0001 g of hydroxyfasudil hydrochloride per 100 mL can be prepared.

The present invention is not limited to the embodiments and examples described above. Various modifications can be made within the scope of the claims, and embodiments obtainable by combining technical means disclosed in different embodiments are also included in the technical scope of the present invention. All the academic literature and patent literature described in this description are incorporated herein by reference.

## Claims

1. A prophylactic or therapeutic agent for axial myopia comprising a Rho kinase inhibitor as an active ingredient.

2. The prophylactic or therapeutic agent for axial myopia according to claim 1, wherein the Rho kinase inhibitor is a compound selected from the group consisting of (R)-(+)-trans-N-(4-pyridyl)-4-(1-aminoethyl)-cyclohexanecarboxamide, 1-(5-isoquinolinesulfonyl)homopiperazine, 1-(1-hydroxy-5-isoquinolinesulfonyl)homopiperazine, (S)-(+)-2-methyl-1-[(4-methyl-5-isoquinolinyl)sulfonyl]-homopiperazine, (S)-(+)-4-glycyl-2-methyl-1-[(4-methyl-5-isoquinolinyl)-sulfonyl]homopiperazine, N-(4-pyridyl)-N'-(2,4,6-trichlorophenyl)urea, and 3-(4-pyridyl)-1H-indole, or a salt thereof.

3. The prophylactic or therapeutic agent for axial myopia according to claim 1 or 2, which is for ophthalmic instillation.

4. The prophylactic or therapeutic agent for axial myopia according to claim 1 or 2, which is for intravitreal administration.
